# EUROPEAN PATENT APPLICATION

(11) **EP 1 078 908 A1**
(43) Date of publication of application: **28.02.2001**
(21) Application number: 00117999.3
(22) Date of filing: 22.08.2000
(51) Int. Cl.: C07C 45/38, C07C 47/544

(54) **Process for producing dialdehyde**

(30) Priority: 23.08.1999 JP 23621499; 19.04.2000 JP 2000117760
(71) Applicant: Daicel Chemical Industries, Ltd., Osaka 590-8501 (JP)
(72) Inventor: Hirai, Naruhisa, Himeji-shi, Hyogo 671-1226 (JP)
(74) Representative: Grünecker, Kinkeldey, Stockmair & Schwanhäusser Anwaltssozietät

(57) **Abstract**

A process produces a dialdehyde. In the process, a 1,4-diol, 1,5-diol, or 1,6-diol is allowed to react with oxygen in the presence of (i) a ruthenium catalyst or of (ii) a metallic catalyst and water to yield a corresponding dialdehyde. A reaction is performed, for example, under neutral or acidic conditions. Water may be added to a reaction system, or a reaction may be performed without removing water out of the reaction system, which water is by-produced during the reaction. The reaction system includes, for example, about 0.01 to 10 moles of water relative to 1 mole of the material diol. The invented process can produce, for example, o-phthalaldehyde from o-benzenedimethanol in a high yield. Thus, a 1,4-diol, 1,5-diol, or 1,6-diol can be oxidized with oxygen to efficiently yield a corresponding dialdehyde.

## Description

### BACKGROUND OF THE INVENTION

### 1. Field of the Invention

The present invention relates to a process for producing a dialdehyde. Specifically, the present invention relates to a process for efficiently producing a corresponding dialdehyde by allowing a 1,4-diol, a 1,5-diol, or a 1,6-diol to react with oxygen.

### 2. Description of the Related Art

As processes for producing a corresponding aldehyde from a primary alcohol, a process of oxidizing the primary alcohol with chromic acid using, for example, pyridinium chlorochromate as an oxidizing agent, and a process using active manganese dioxide as an oxidizing agent are known. However, these processes require large amounts of metallic compounds and are economically disadvantageous. In addition, these processes require complicated after-treatments and are not industrially practical. A process of oxidizing a primary alcohol with dimethyl sulfoxide to yield a corresponding aldehyde is also known. However, this process invites malodor problems and cannot be employed in commercial mass production.

Alternatively, a process of converting an alcohol into an aldehyde using oxygen as an oxidizing agent is proposed, from the viewpoints of cost efficiency and environmental protection. However, few processes have been known in which a dialdehyde is efficiently produced from a 1,4-diol, a 1,5-diol, or a 1,6-diol. In addition, when a 1,4-diol, a 1,5-diol or a 1,6-diol is oxidized, a reaction intermediate (a monoaldehyde) is liable to form a 5-membered ring, a 6-membered ring, or a 7-membered ring, respectively, to thereby yield large amounts of by-products having the aforementioned cyclic structure. The target dialdehyde cannot be significantly obtained in a high yield.

### SUMMARY OF THE INVENTION

Accordingly, an object of the invention is to provide a process for efficiently producing a corresponding dialdehyde by oxidizing a 1,4-diol, a 1,5-diol, or a 1,6-diol with oxygen.

Another object of the invention is to provide a process for producing a corresponding dialdehyde in a good yield by oxidizing a 1,4-diol, a 1,5-diol, or a 1,6-diol with oxygen while minimizing the formation of by-products.

After intensive investigations to achieve the objects, the present inventors found that a corresponding dialdehyde can be efficiently formed by allowing a 1,4-diol, a 1,5-diol, or a 1,6-diol to react with oxygen in the presence of a ruthenium catalyst or in the presence of a metallic catalyst and water. The present invention has been accomplished based on these findings.

Specifically, the invention provides a process for producing a dialdehyde. This process includes the step of allowing a 1,4-diol, a 1,5-diol, or a 1,6-diol to react with oxygen (i) in the presence of a ruthenium catalyst or (ii) in the presence of a metallic catalyst and water to yield a corresponding dialdehyde.

The 1,4-diol, 1,5-diol, or 1,6-diol includes a compound shown by the following formula (1), (2), or (3) : 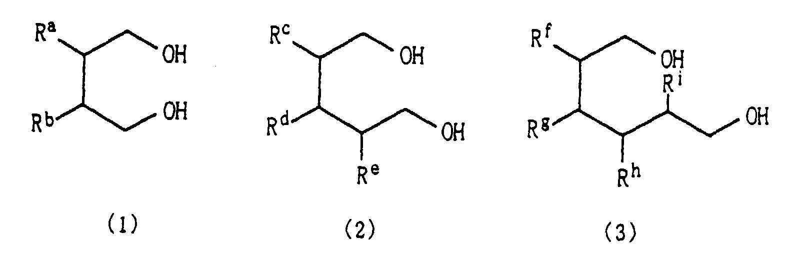 wherein each of R^{a} and R^{b} in the formula (1) is, identical to or different from each other, a hydrogen atom, a hydroxyl group which may be protected by a protective group, a hydrocarbon group, or a heterocyclic group, where R^{a} and R^{b} may be combined to form a double bond, or to form a ring with the adjacent two carbon atoms; each of R^{c}, R^{d}, and R^{e} in the formula (2) is, identical to or different from one another, a hydrogen atom, a hydroxyl group which may be protected by a protective group, a hydrocarbon group, or a heterocyclic group, where at least two of R^{c}, R^{d}, and R^{e} may be combined to form a double bond, or to form a ring with the adjacent carbon atoms; and each of R^{f}, R^{g}, R^{h}, and Rⁱ in the formula (3) is, identical to or different from one another, a hydrogen atom, a hydroxyl group which may be protected by a protective group, a hydrocarbon group, or a heterocyclic group, where at least two of R^{f}, R^{g}, R^{h}, and Rⁱ may be combined to form a double bond, or to form a ring with the adjacent carbon atoms.

In the formula (1), R^{a} and R^{b} may be combined to form, with the adjacent carbon atoms, an aromatic carbon ring or an aromatic heterocyclic ring which may have a substituent on a ring. The compound shown by the formula (1) includes, for example, o-benzenedimethanol.

As the ruthenium catalyst, at least one selected from a metallic ruthenium, a ruthenium oxide, a perruthenic acid or its salt, and a ruthenium complex may be employed. The ruthenium catalyst may be a carrier-supported ruthenium catalyst including a catalytic component supported on a carrier.

As the metallic catalyst, at least one catalyst selected from a catalyst containing an element of Groups 5 to 11 of the Periodic Table of Elements, and a catalyst comprising a heteropolyacid or its salt may be employed. The metallic catalyst may be a carrier-supported metallic catalyst including a catalytic component supported on a carrier.

According to the invented process, a reaction is preferably performed, for example, under neutral or acidic conditions. In the process, water may be added to a reaction system, or a reaction may be performed without removing water out of the reaction system, which water is by-produced during the reaction. The reaction system may include 0.01 to 10 moles of water relative to 1 mole of the material dial.

In this specification, the 1,4-diol, 1,5-diol, or 1,6-diol used as a reaction material (reactant) may be simply referred to as "substrate".

### DESCRIPTION OF THE PREFERRED EMBODIMENTS

### [1,4-Diol, 1,5-Diol or 1,6-Diol]

The 1,4-diols, 1,5-diols, and 1,6-diols for use as reactants (substrates) in the invention are 1,4-diols, 1,5-diols, and 1,6-diols each having primary hydroxyl groups (hydroxyl groups constituting a primary alcohol) at both ends. The term "1,4-diol" as used herein means a diol having two hydroxyl groups with the interposition of four carbon atoms (a carbon chain having four carbon atoms); the term "1,5-diol" means a diol having two hydroxyl groups with the interposition of five carbon atoms (a carbon chain having five carbon atoms); and the term "1,6-diol" means a dial having two hydroxyl groups with the interposition of six carbon atoms (a carbon chain having six carbon atoms) . The aforementioned carbon atoms may be carbon atoms constituting an aromatic ring or a non-aromatic ring. Such diols include, for example, chain diols, and cyclic compounds each having hydroxyalkyl groups (hydroxymethyl group, 2-hydroxyethyl group, or 3-hydroxypropyl group) bonded to two carbon atoms constituting a ring. Each of these substrates can be used alone or in combination.

The substrates include, but are not limited to, 1,4-diols of the formula (1), 1,5-diols of the formula (2), and 1,6-diols of the formula (3).

In the formulae, known or conventional protective groups in the field of organic synthesis can be used as the protective group for hydroxyl group. Hydrocarbon groups in R^{a}, R^{b}, R^{c}, R^{d}, R^{e}, R^{f}, R^{g}, R^{h}, and Rⁱ in the formulae include aliphatic hydrocarbon groups, alicyclic hydrocarbon groups, aromatic hydrocarbon groups, and groups formed from these groups combined with each other. Such aliphatic hydrocarbon groups include, but are not limited to, methyl, ethyl, propyl, isopropyl, butyl, isobutyl, s-butyl, t-butyl, pentyl, hexyl, decyl, dodecyl, and other alkyl groups each having about 1 to 20, and preferably about 1 to 10 carbon atoms; vinyl, allyl, 1-butenyl, and other alkenyl groups each having about 2 to 20, and preferably about 2 to 10 carbon atoms; and ethynyl, propynyl, and other alkynyl groups each having about 2 to 20, and preferably about 2 to 10 carbon atoms.

The alicyclic hydrocarbon groups include, but are not limited to, cyclopropyl, cyclobutyl, cyclopentyl, cyclohexyl, cyclooctyl, and other cycloalkyl groups each having about 3 to 20, preferably about 3 to 15, and more preferably about 5 to 8 members; and cyclopentenyl, cyclohexenyl, and other cycloalkenyl groups each having about 3 to 20, preferably about 3 to 15, and more preferably about 5 to 8 members. The aromatic hydrocarbon groups include, for example, phenyl, naphthyl, and other aromatic hydrocarbon groups each having about 6 to 14, and preferably about 6 to 10 carbon atoms.

Hydrocarbon groups formed from an aliphatic hydrocarbon group and an alicyclic hydrocarbon group combined with each other include, for example, cyclopentylmethyl, cyclohexylmethyl, 2-cyclohexylethyl, and other cycloalkyl-alkyl groups (e.g., C₃-C₂₀ cycloalkyl-C₁-C₄ alkyl groups). Hydrocarbon groups formed from an aliphatic hydrocarbon group and an aromatic hydrocarbon group combined with each other include, for example, aralkyl groups (e.g., C₇-C₁₈ aralkyl groups), and alkyl-substituted aryl groups (e.g., phenyl group or naphthyl group substituted with about one to four C₁-C₄ alkyl groups).

Preferred hydrocarbon groups include, for example, C₁-C₁₀ alkyl groups, C₂-C₁₀ alkenyl group, C₂-C₁₀ alkynyl groups, C₃-C₁₅ cycloalkyl groups, C₆-C₁₀ aromatic hydrocarbon groups, C₃-C₁₅ cycloalkyl-C₁-C₄ alkyl groups, and C₇-C₁₄ aralkyl groups.

The hydrocarbon groups may have a variety of substituents. Such substituents include, but are not limited to, halogen atoms, oxo group, hydroxyl group, substituted oxy groups (e.g., alkoxy groups, aryloxy groups, aralkyloxy groups, and acyloxy groups), carboxyl group, substituted oxycarbonyl groups, substituted or unsubstituted carbamoyl groups, cyano group, nitro group, substituted or unsubstituted amino groups, and heterocyclic groups.

Heterocyclic rings constituting the heterocyclic groups in R^{a}, R^{b}, R^{c}, R^{d}, R^{e}, R^{f}, R^{g}, R^{h}, and Rⁱ include aromatic heterocyclic rings and non-aromatic heterocyclic rings. Such heterocyclic rings include, but are not limited to, heterocyclic rings each containing an oxygen atom as a heteroatom (e.g., furan, tetrahydrofuran, oxazole, isoxazole, and other 5-membered rings, 4-oxo-4H-pyran, tetrahydropyran, morpholine, and other 6-membered rings, benzofuran, isobenzofuran, 4-oxo-4H-chromene, chroman, isochroman, and other condensed rings), heterocyclic rings each containing a sulfur atom as a heteroatom (e.g., thiophene, thiazole, isothiazole, thiadiazole, and other 5-membered rings, 4-oxo-4H-thiopyran, and other 6-membered rings, benzothiophene and other condensed rings), heterocyclic rings each containing a nitrogen atom as a heteroatom (e.g., pyrrole, pyrrolidine, pyrazole, imidazole, triazole, and other 5-membered rings, pyridine, pyridazine, pyrimidine, pyrazine, piperidine, piperazine, and other 6-membered rings, indole, indoline, quinoline, acridine, naphthyridine, quinazoline, purine, and other condensed rings). These heterocyclic groups may have substituents. Such substituents include, for example, the substituents which the hydrocarbon groups may have, as well as alkyl groups (e.g., methyl, ethyl, and other C₁-C₄ alkyl groups), cycloalkyl groups, and aryl groups (e.g., phenyl, and naphthyl groups).

The groups R^{a} and R^{b}; at least two groups of R^{c}, R^{d}, and R^{e}; or at least two groups of R^{f}, R^{g}, R^{h}, and Rⁱ may be combined to form a ring with the adjacent carbon atoms. Such rings include, but are not limited to, benzene, naphthalene, anthracene, and other aromatic carbon rings; cyclobutane, cyclopentane, cyclohexane, cyclohexene, cyclooctane, cyclododecane, and other non-aromatic carbon rings (cycloalkane rings, cycloalkene rings, or bridged carbon rings) each having about 3 to 20 members (preferably about 3 to 15 members, and more preferably about 5 to 12 members); furan, thiophene, pyrrole, pyridine, quinoline, and other aromatic heterocyclic rings; oxolane, oxane, azolidine, perhydroazine, thiolane, thiane, and other non-aromatic heterocyclic rings (especially, non-aromatic heterocyclic rings each containing an oxygen atom, a nitrogen atom, or a sulfur atom) each having about 3 to 20 members (preferably about 3 to 12 members, and more preferably about 3 to 8 members). These rings may have substituents similar to the substituents which the heterocyclic groups may have, and other rings (non-aromatic or aromatic rings) may be condensed to these rings.

Preferred groups R^{a}, R^{b}, R^{c}, R^{d}, R^{e}, R^{f}, R^{g}, R^{h}, and Rⁱ include, for example, hydrogen atom, hydroxyl group which may be protected by a protective group, C₁-C₁₀ alkyl groups, C₂-C₁₀ alkenyl groups, C₂-C₁₀ alkynyl groups, C₃-C₁₅ cycloalkyl groups, C₆-C₁₀ aromatic hydrocarbon groups, C₃-C₁₂ cycloalkyl-C₁-C₄ alkyl groups, and C₇-C₁₄ aralkyl groups. Alternatively, the groups R^{a} and R^{b}; at least two groups of R^{c}, R^{d}, and R^{e}; or at least two groups of R^{f}, R^{g}, R^{h}, and Rⁱ are preferably combined to form a double bond, or to form, with the adjacent carbon atoms, an aromatic carbon ring, an aromatic heterocyclic ring, or a non-aromatic carbon ring or non-aromatic heterocyclic ring each having about 3 to 20 members. The above groups are typically preferably combined to form an aromatic carbon ring or an aromatic heterocyclic ring.

Especially preferred substrates for use in the invention are compounds of the formula (1) in which R^{a} and R^{b} are combined to form, with the adjacent carbon atoms, an aromatic carbon ring or aromatic heterocyclic ring which may have a substituent on its ring.

Typical 1,4-diols, 1,5-diols, and 1,6-diols of the formulae (1), (2), and (3) include, but are not limited to, 1,4-butanediol, 1,5-pentanediol, 1,6-hexanediol, polyhydric alcohols each having a secondary hydroxyl group protected by a protective group (e.g., derivatives of xylitol, sorbitol, mannitol, and other alditols each having a protected secondary hydroxyl group), and other chain diols; o-benzenedimethanol (o-xylylene alcohol), 2,3-naphthalenedimethanol, 1,8-naphthalenedimethanol, 1-(2-hydroxyethyl)-8-hydroxymethylnaphthalene, 2-(2-hydroxymethylphenyl)ethanol, 1,2-cyclohexanedimethanol, 3,4-furandimethanol, 3,4-thiophenedimethanol, 2,3-pyridinedimethanol, 2,3-quinolinedimethanol, and other aromatic or non-aromatic cyclic compounds each having hydroxyalkyl groups (hydroxymethyl group, 2-hydroxyethyl group, or 3-hydroxypropyl group) bonded to two carbon atoms (e.g., adjacent carbon atoms) constituting a ring. These cyclic compounds may have a substituent on the rings.

### [Ruthenium Catalyst]

The ruthenium catalyst includes elementary ruthenium and compounds containing a ruthenium atom. Such ruthenium catalysts include, but are not limited to, metallic ruthenium, ruthenium oxide, ruthenium sulfide, ruthenium hydroxide, ruthenium fluoride, ruthenium chloride, ruthenium bromide, ruthenium iodide, ruthenium sulfate, ruthenic acid or its salts (e.g., ammonium ruthenate), perruthenic acid or its salts (e.g., tetrapropylammonium perruthenate), inorganic ruthenium complexes [e.g., ruthenium hydroxyhalides such as ruthenium hydroxychloride; hexaammineruthenium halides such as hexaammineruthenium chloride; ruthenium nitrosyl, hexahaloruthenic acid or salts thereof such as sodium hexachlororuthenate], and other inorganic ruthenium compounds; ruthenium cyanide, organic ruthenium complexes [e.g., dodecacarbonyltriruthenium(0), dicarbonyltris(triphenylphosphine)ruthenium(II), diacetatodicarbonylbis(triphenylphosphine)ruthenium(II), dichlorotris(triphenylphosphine)ruthenium(II), dihydridotetrakis(triphenylphosphine)ruthenium(II), dichlorobis(acetonitrile)bis(triphenylphosphine)ruthenium( II), and ruthenocene], and other organic ruthenium compounds.

The ruthenium may have any valency ranging from 0 to 8, and preferably has a valency ranging from 0 to 4. Preferred ruthenium catalysts include metallic ruthenium, perruthenic acid or salts thereof, and ruthenium complexes, of which metallic ruthenium and ruthenium complexes are typically preferred. Each of these ruthenium catalysts can be used alone or in combination.

The ruthenium catalyst may be a carrier-supported ruthenium catalyst comprising a catalytic component (metallic ruthenium or a ruthenium compound) supported on a carrier. Especially when metallic ruthenium is employed as the catalytic component, the catalytic activity of the catalyst can be markedly enhanced by supporting the catalytic component on a carrier. Such carriers include conventional carriers for use in supporting catalysts, such as activated carbon, silica, alumina, silica-alumina, zeolite, titania, magnesia, diatomaceous earth, kaolin, and other inorganic carriers, styrene-divinylbenzene copolymers, and other organic carriers. Among them, activated carbon and alumina are typically preferred for high catalytic activities. Such activated carbons include activated carbons derived from various materials such as plants, minerals, and resins. These activated carbons may be activated with either a gas or a chemical agent. The carrier has a specific surface area of, for example, about 10 to 3000 m²/g, and preferably about 50 to 3000 m²/g.

The proportion of the catalytic component in the carrier-supported ruthenium catalyst is, for example, about 0.1% to 50% by weight, preferably about 1% to 20% by weight, and more preferably about 2% to 10% by weight, relative to the weight of the carrier. The catalyst can be prepared in a conventional manner such as impregnation, precipitation, or ion exchange.

The amount of the ruthenium catalyst is, for example, about 0.001 to 1 mole, preferably about 0.01 to 0.6 mole, and more preferably about 0.02 to 0.4 mole in terms of a ruthenium compound (or metallic ruthenium) relative to 1 mole of the substrate diol.

### [Metallic Catalyst]

The metallic catalyst has only to be a metal or a metallic compound that exhibits catalytic activities upon oxidation of an alcohol with oxygen into an aldehyde. Such metallic catalysts include, but are not limited to, ruthenium catalysts, palladium catalysts, platinum catalysts, and other noble metal catalysts, chromium catalysts, iron catalysts, manganese catalysts, copper catalysts, and other catalysts each containing an element of Groups 5 to 11 of the Periodic Table of Elements; and catalysts comprising a heteropolyacid or its salt. Of these catalysts, ruthenium catalysts, or catalysts comprising a heteropolyacid or its salt are preferred. Such ruthenium catalysts are the same as stated above.

As the heteropolyacid, condensates of various oxoacids each having two or more different central ions can be employed. Typical heteropolyacids are composed of an oxoacid ion of an element such as P, As, Sn, Si, Ti, or Zr, and an oxoacid of an element such as V, Mo or W. Among them, heteropolyacids each containing at least one selected from P, Si, V, Mo, and W are preferred, of which heteropolyacids each containing P and at least one selected from V and Mo are especially preferred.

Heteropolyacid anions for constituting heteropolyacids or salts thereof may have various compositions, of which a composition shown by the formula XM₁₂O₄₀ is preferred, wherein X is, for example, Si, P, or another element; and M is Mo, W, V, or another element. The element M may be one element or a combination of two or more elements. Illustrative heteropolyacid anions having such a composition include anions of, for example, phosphomolybdic acid, phosphotungstic acid, silicomolybdic acid, silicotungstic acid, and phosphovanadomolybdic acid.

Salts of heteropolyacids include a variety of compounds in which at least part of hydrogen atoms corresponding to the cation of a heteropolyacid are substituted with other cations. Such substitutable cations include, but are not limited to, ammonium (e.g., NH₄), alkali metals (e.g., Cs, Rb, K, Na, and Li), and alkaline earth metals (e.g., Ba, Sr, Ca, and Mg). Among them, salts of heteropolyacids in which part of hydrogen atoms of the heteropolyacid is substituted with NH₄, i.e., the cation component is composed of H and NH₄, are preferred to attain high catalytic activity and stability. In this case, the ratio of NH₄ to H is, for example such that NH₄/H (by mole) is about 0.1 to 10, preferably about 0.2 to 8, and more preferably about 0.3 to 5.

Of these heteropolyacids or salts thereof, phosphovanadomolybdic acids of the following formula or salts thereof can be advantageously employed:

A₃₊ₙ[PVₙMo₁₂₋ₙO₄₀]

wherein A is a heteropolyacid cation; and n denotes an integer of 1 to 10.

The cation A includes hydrogen atom and the aforementioned cations. The value n can be appropriately selected with reference to oxidative power and stability of the catalyst, and is preferably about 4 to 10 (e.g., about 4 to 8), and more preferably about 5 to 8. When the heteropolyacid cation is composed of H and another cation (e.g., NH₄), the value n ranges 4 to 10 in many cases. Each of these heteropolyacids and salts thereof can be used alone or in combination.

The metallic catalyst may be a carrier-supported metallic catalyst comprising a catalytic component supported on a carrier. The catalytic activity of the metallic catalyst will be often enhanced by supporting the catalytic component on a carrier. Such carriers include those mentioned above. The proportion of the catalytic component in the carrier-supported metallic catalyst is, for example, about 0.1% to 50% by weight, and preferably about 1% to 20% by weight relative to the weight of the carrier. The catalyst can be prepared in a conventional manner such as impregnation, precipitation, or ion exchange.

The amount of the metallic catalyst (the catalytic component when the metallic catalyst is a carrier-supported metallic catalyst) is, for example, about 0.001 to 1 mole, preferably about 0.01 to 0.6 mole, and more preferably about 0.02 to 0.4 mole relative to 1 mole of the substrate diol. Each of these metallic catalysts can be used alone or in combination.

In the invention, an acid (an acid other than a heteropolyacid in the above embodiment (ii)) can be used in combination with the catalyst (the ruthenium catalyst or metallic catalyst). Some combinations of the catalyst (e.g., a heteropolyacid or its salt) with an acid can increase the catalytic activity. Such acids include, but are not limited to, sulfuric acid, nitric acid, hydrogen halides (hydrogen fluoride, hydrogen chloride, hydrogen bromide, hydrogen iodide, and corresponding hydrohalogen acids), phosphoric acid, boric acid, and other inorganic acids; formic acid, acetic acid, monochloroacetic acid, trichloroacetic acid, trifluoroacetic acid, benzoic acid, and other carboxylic acids, methanesulfonic acid, trifluoromethanesulfonic acid, benzenesulfonic acid, p-toluenesulfonic acid, naphthalenesulfonic acid, and other sulfonic acids, and other organic acids; and sulfonic acid type ion exchange resins, and other strongly acidic ion exchange resins. Each of these acids can be used alone or in combination.

Of these acids, preferred are protonic acids having pKa of 3 or less (e.g., about -14 to 3), and typically 0 or less (e.g., -14 to 0) in an aqueous solution at 25°C. Such preferred acids include, for example, sulfuric acid, nitric acid, hydrogen halides including hydrohalogen acids, phosphoric acid, and other inorganic acids; sulfonic acids; and trichloroacetic acid, and other haloalkane acids.

The proportion of the acid is not critical and is, for example, about 0.01% to 250% by mole, preferably about 0.1% to 200% by mole, and more preferably about 2% to 50% by mole, relative to the substrate. In this connection, acetic acid and other carboxylic acids and the like can be employed as reaction solvents, as described below.

In the invention, a co-catalyst such as hydroquinone or another dioxybenzene or its oxide (oxidant) may exist in a reaction system, but such a co-catalyst is not always required. A base may proceed a reaction in some oxidation reactions, but the reaction according to the invention is preferably performed under neutral or acidic conditions. If the substrate 1,4-diol, 1,5-diol or 1,6-diol is allowed to react with oxygen under basic conditions, the below-described lactones (γ-lactone, δ-lactone, or ε-lactone) are liable to be by-produced, which may markedly deteriorate the selectivity of the target dialdehyde. For example, if o-benzenedimethanol is oxidized under basic conditions, the amount of phthalide is markedly increased and the selectivity of the target o-phthalaldehyde is decreased.

### [Water]

An important feature in the embodiment (ii) of the invention is that the substrate is allowed to react with oxygen in the presence of water. The water may be either liquid or gaseous. The water include not only free water but also crystal water contained in, for example, the catalyst used. To the reaction system, the water may be added at the beginning of the reaction or may be continuously or intermittently added during the reaction. Alternatively, water by-produced in the reaction may be held in the reaction system without removing it out of the system. Among these procedures, a procedure of adding water to the reaction system at the beginning of, or during the reaction.

If the reaction is performed while removing water by-produced in the reaction out of the system without adding water to the system, the amount of a dehydrated condensate (an acetal compound) between the substrate diol and a reaction intermediate or its isomer (e.g., a compound in which one of two hydroxymethyl groups is oxidized into a formyl group, or its isomer cyclic hemiacetal) is increased to thereby greatly decrease the yield of the target dialdehyde. Accordingly, the removal of water out of the reaction system using a Dean-Stark device, decanter, or other water separators is not preferred.

The amount of water in the reaction system is not critical and is, for example, about 0.01 to 10 moles, preferably about 0.01 to 5 moles, and more preferably about 0.05 to 4 moles, relative to 1 mole of the substrate. Especially, the amount of water should be preferably 2.01 moles or more (e.g., about 2.01 to 10 moles, and particularly about 2.05 to 10 moles) for a higher yield.

### [Oxygen]

As the oxygen, any of molecular oxygen and nascent oxygen can be used. Such molecular oxygen includes, but is not limited to, pure oxygen, and oxygen diluted with an inert gas such as nitrogen, helium, argon or carbon dioxide. Air is preferably used as the molecular oxygen from the viewpoints of operating property and safety, as well as cost efficiency.

The proportion of the oxygen can be appropriately selected according to the type of the substrate, and generally is, for example, 1 mole or more, preferably about 1 to 100 moles, and more preferably about 2 to 50 moles, relative to 1 mole of the substrate. The oxygen is often used in excess moles with respect to the substrate.

### [Reaction]

The reaction can be performed either in a liquid phase or in a gaseous phase. In a liquid phase reaction, the reaction is generally performed in an organic solvent. Such organic solvents include, but are not limited to, benzene, toluene, xylene, ethylbenzene, trifluoromethylbenzene (trifluorotoluene), chlorobenzene, anisole, benzonitrile, nitrobenzene, ethyl benzoate, and other benzene derivatives whose benzene ring may be substituted with, for example, a halogen atom, an alkyl group, a haloalkyl group, an alkoxy group, a cyano group, a nitro group, or a substituted oxycarbonyl group; hexane, heptane, octane, and other aliphatic hydrocarbons; cyclohexane, and other alicyclic hydrocarbons; carbon tetrachloride, chloroform, dichloromethane, 1,2-dichloroethane, and other haloalkanes; acetone, methyl ethyl ketone, and other ketones; methyl acetate, ethyl acetate, isopropyl acetate, butyl acetate, and other esters; N,N-dimethylformamide, N,N-dimethylacetamide, and other amides; acetonitrile, propionitrile, and other nitriles; diethyl ether, dibutyl ether, dimethoxyethane, dioxane, tetrahydrofuran, and other chain or cyclic ethers; and acetic acid, and other organic acids. Preferred solvents are benzene, toluene, trifluoromethylbenzene, and other benzene derivatives mentioned above, and butyl acetate, and other esters. In addition, solvents miscible with water can dissolve water and are also preferred. Such miscible solvents include acetic acid, and other organic acids, acetonitrile, and other nitriles, N,N-dimethylformamide, and other amides, and other polar solvents. Each of these solvents can be used alone or in combination.

A reaction temperature can be appropriately selected according to, for example, the type of the substrate, and is, for example, about 0°C to 200°C, preferably about 10°C to 150°C, and more preferably about 30°C to 120°C. The reaction may be performed at atmospheric pressure or under a pressure (under a load) [e.g., at a pressure of 1 to 100 atm (0.1 to 10 MPa)]. The reaction can be conducted in a batch system, a semi-batch system, a continuous system, or any other systems.

According to the invented process, two hydroxymethyl groups at terminals are oxidized upon reaction to yield a corresponding dialdehyde even under mild conditions. For example, the diol of the formula (1), (2), or (3) can respectively yield a corresponding dialdehyde shown by the following formula (4), (5), or (6): 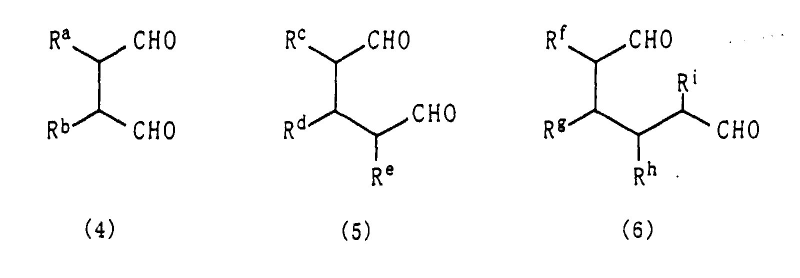 wherein R^{a}, R^{b}, R^{c}, R^{d}, R^{e}, R^{f}, R^{g}, R^{h}, and Rⁱ have the same meanings as defined above.

After the completion of the reaction, reaction products can be easily separated and purified in a conventional manner such as filtration, concentration, distillation, extraction, crystallization, recrystallization, column chromatography, and other separation means, and combinations of these separation means.

When the 1,4-diol, 1,5-diol or 1,6-diol is oxidized, an intermediate (a compound in which one of two hydroxymethyl groups is oxidized into a formyl group; a monoaldehyde) is liable to form a 5-membered ring, a 6-membered ring, or a 7-membered ring, respectively, and a compound having such a ring structure is liable to be by-produced. For example, the diol of the formula (1), (2), or (3) can respectively yield a lactone shown by the following formula (7), (8), or (9). These lactones are supposed to be formed by isomerizing the intermediate product into a corresponding cyclic hemiacetal compound and oxidizing the hemiacetal compound. Acetal compounds having a cyclic structure are also liable to be formed as by-products. For example, the compound of the formula (1) can yield an acetal compound shown by the following formula (10a). Likewise, the diol of the formula (2) or (3) can yield a corresponding acetal compound having a 6-membered or 7-membered ring structure. These acetal compounds are supposed to be formed upon dehydration-condensation of the substrate diol with the monoaldehyde or the cyclic hemiacetal compound. In addition, a cyclic compound as an acetal compound of the material diol with the target dialdehyde is also liable to be by-produced. For example, the diol of the formula (1) can yield acetal compounds of the following formula (10b) and (10c): 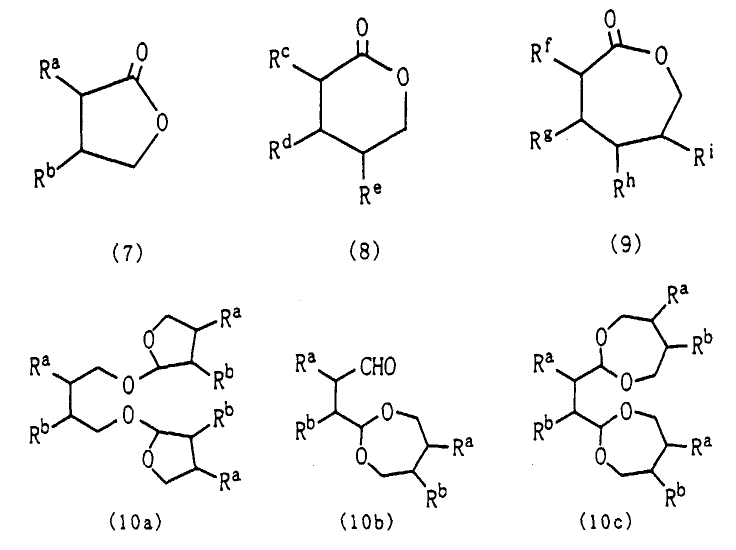 wherein R^{a}, R^{b}, R^{c}, R^{d}, R^{e}, R^{f}, R^{g}, R^{h}, and Rⁱ have the same meanings as defined above.

The aforementioned reaction will be illustrated in further detail by taking o-benzenedimethanol as example of the substrate. As shown in the following reaction process chart, o-benzenedimethanol (11) is allowed to react with oxygen in the presence of a metallic catalyst, and a monoaldehyde (o-hydroxymethylbenzaldehyde) of the formula (13) is initially formed, and the monoaldehyde is then further oxidized to yield a target compound dialdehyde (phthalaldehyde) of the formula (16). Part of the monoaldehyde (13) is isomerized in a system to yield a cyclic hemiacetal compound of the formula (12). When the cyclic hemiacetal compound (12) is further oxidized, a lactone compound (phthalide) of the formula (15) is formed.

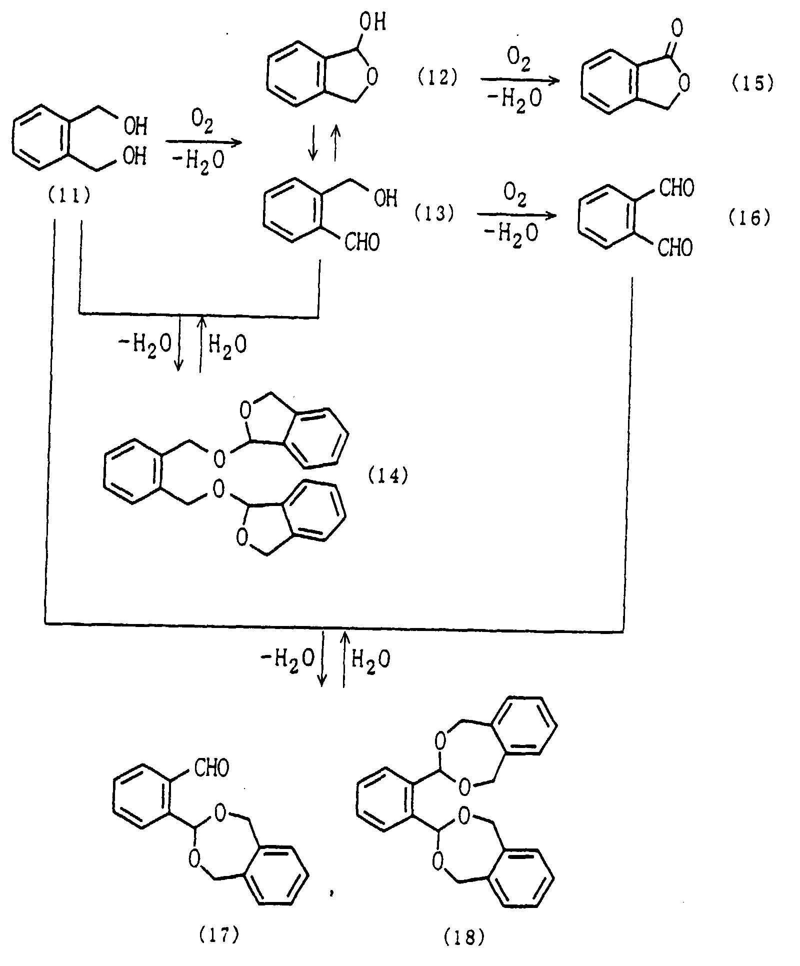

Separately, the cyclic hemiacetal compound (12) and/or the monoaldehyde (13) undergoes dehydration-condensation with the material o-benzenedimethanol (11) to yield an acetal compound of the formula (14). When water exists in the system, the acetal compound (14) undergoes a reverse reaction to yield the cyclic hemiacetal compound (12) and/or the monoaldehyde (13) and the material o-benzenedimethanol (11). The material o-benzenedimethanol (11) and the target dialdehyde (16) undergo dehydration-condensation to yield a cyclic acetal compound of the formula (17) and/or the formula (18). These cyclic acetal compounds (17) and (18) undergo a reverse reaction to yield the material o-benzenedimethanol (11) and the target dialdehyde (16). These reactions are reversible reactions. Accordingly, when water by-produced in the reaction is removed out of the reaction system, the reaction proceeds in such a direction as to yield the acetal compounds (14), (17), and (18) to greatly deteriorate the yield of the target dialdehyde (16). In contrast, according to the embodiment (ii) of the invention, the reaction is performed in the presence of water, and proceeds in such a direction as to consume the acetal compounds (14), (17), and (18). Accordingly, the formation reaction of the target dialdehyde (16) can very advantageously proceed. In addition, according to the invented process, by-production of the lactones (e.g., the compounds of the formulae (7), (8), and (9)) is minimized.

The lactone can be converted into a corresponding diol (material diol) or a cyclic hemiacetal (intermediate of the target compound) by a conventional reducing process such as a reducing process with a metal-hydrogen complex or a catalytic reduction process. The by-produced lactone, if any, can be reduced to yield the diol or cyclic hemiacetal and can be recycled to the reaction system. Likewise, the acetal compound can be converted into, for example, the material diol and/or cyclic hemiacetal (intermediate of the target compound) by hydrolysis. The acetal compound, if it is by-produced, can be recycled to the reaction system as intact or after hydrolysis.

A preferred embodiment of the invention includes a step of recycling by-products such as lactones by-produced in the reaction to the reaction system as intact or after reducing or hydrolyzing the by-products to yield, for example, the material diol.

As thus described, the invented process employs a specific catalyst and allows oxygen to oxidize a 1,4-diol, 1,5-diol, or 1,6-diol to efficiently yield a corresponding dialdehyde.

In addition, the invented process oxidizes a 1,4-diol, 1,5-diol, or 1,6-diol under specific conditions and can minimize the formation of by-products each having a cyclic structure, and a corresponding dialdehyde can be produced in a good yield.

The present invention will now be illustrated in further detail with reference to several examples below, which are not intended to limit the scope of the invention.

### EXAMPLE 1

A mixture of 2 mmol (in terms of ruthenium) of a 5% by weight Ru/C, 10 mmol of o-benzenedimethanol (o-xylylene alcohol), and 10 ml of toluene was stirred at 60°C in an oxygen atmosphere (1 atm) for 4 hours. A gas chromatographic analysis of products revealed that o-phthalaldehyde and phthalide (1-phthalanone) were formed in yields of 50.3% and 25.1%, respectively.

### EXAMPLE 2

A mixture of 2 mmol (in terms of ruthenium) of a 5% by weight Ru/C, 10 mmol of o-benzenedimethanol (o-xylylene alcohol), and 10 ml of acetic acid was stirred at 60°C in an oxygen atmosphere (1 atm) for 4 hours. A gas chromatographic analysis of products revealed that o-phthalaldehyde and phthalide (1-phthalanone) were formed in yields of 34.8% and 30.2%, respectively.

### EXAMPLE 3

A mixture of 0.16 g (0.8 mmol) of RuO₂.H₂O, 1.11 g (8 mmol) of o-benzenedimethanol (o-xylylene alcohol), and 10 g of toluene was stirred at 100°C in an oxygen atmosphere (1 atm = 0.101 MPa) for 8 hours. A gas chromatographic analysis of products revealed that o-phthalaldehyde and phthalide (1-phthalanone) were formed in yields of 66.0% and 17.0%, respectively.

### EXAMPLE 4

A mixture of 0.16 g (0.8 mmol) of RuO₂.H₂O, 1.11 g (8 mmol) of o-benzenedimethanol, and 10 g of n-butyl acetate was stirred at 100°C in an oxygen atmosphere (1 atm = 0.101 MPa) for 10 hours. A gas chromatographic analysis of products revealed that o-phthalaldehyde and phthalide were formed in yields of 67.1% and 14.8%, respectively.

### EXAMPLE 5

A mixture of 1.62 g (0.8 mmol in terms of ruthenium) of a 5% by weight Ru/C, 1.11 g (8 mmol) of o-benzenedimethanol, 10 g of N,N-dimethylformamide, and 0.48 g of acetic acid was stirred at 100°C in an oxygen atmosphere (1 atm = 0.101 MPa) for 8 hours. A gas chromatographic analysis of products revealed that o-phthalaldehyde and phthalide were formed in yields of 54.1% and 15.8%, respectively.

### EXAMPLE 6

A mixture of 1.62 g (0.8 mmol in terms of ruthenium) of a 5% by weight Ru/alumina, 1.11 g (8 mmol) of o-benzenedimethanol, 10 g of N,N-dimethylformamide, and 0.48 g of acetic acid was stirred at 100°C in an oxygen atmosphere (1 atm = 0.101 MPa) for 8 hours. A gas chromatographic analysis of products revealed that o-phthalaldehyde and phthalide were formed in yields of 56.3% and 15.3%, respectively.

### EXAMPLE 7

In a titanium autoclave, 1.45 g (0.0072 mol) of RuO₂.H₂O, 10.00 g (0.072mol) of o-benzenedimethanol, and 190 g of toluene were placed, and the resulting mixture was stirred at 100°C in an air atmosphere (30 kgf/cm² = 2.94 MPa) for 2 hours. A gas chromatographic analysis of products revealed that o-phthalaldehyde and phthalide were formed in yields of 68.1% and 24.3%, respectively.

### EXAMPLE 8

In a titanium autoclave, 1.45 g (0.0072 mol) of RuO₂.H₂O, 10.00 g (0.072 mol) of o-benzenedimethanol, and 90 g of n-butyl acetate were placed, and the resulting mixture was stirred at 100°C in an air atmosphere (30 kgf/cm² = 2.94 MPa) for 2 hours. A gas chromatographic analysis of products revealed that o-phthalaldehyde and phthalide were formed in yields of 68.3% and 28.6%, respectively.

### EXAMPLE 9

In a titanium autoclave, 0.72 g (0.0036 mol) of RuO₂.H₂O, 10.00 g (0.072 mol) of o-benzenedimethanol, and 90 g of toluene were placed, and the resulting mixture was stirred at 100°C in an air atmosphere (30 kgf/cm² = 2.94 MPa) for 4 hours. A gas chromatographic analysis of products revealed that o-phthalaldehyde and phthalide were formed in yields of 62.3% and 24.3%, respectively.

### EXAMPLE 10

A mixture of 0.35 g of a 10% by weight (NH₄)₅H₆PMo₄V₈O₄₀/C, 0.27 g (2 mmol) of o-benzenedimethanol, 0.07g (4 mmol) of water, 0.04 g (0.4 mmol) of methanesulfonic acid, and 6 g of toluene was stirred at 100°C in an oxygen atmosphere (1 atm = 0.101 MPa) for 15 hours. A gas chromatographic analysis of products revealed that o-phthalaldehyde and phthalide were formed in yields of 72.0% and 1.0%, respectively.

### EXAMPLE 11

The procedure of Example 10 was repeated, except that 0.08 g (0.4 mmol) of p-toluenesulfonic acid was employed instead of methanesulfonic acid to find that o-phthalaldehyde and phthalide were formed in yields of 65.2% and 2.1%, respectively.

Other embodiments and variations will be obvious to those skilled in the art, and this invention is not to be limited to the specific matters stated above.

## Claims

1. A process for producing a dialdehyde, comprising the step of:
allowing a 1,4-diol, a 1,5-diol, or a 1,6-diol to react with oxygen (i) in the presence of a ruthenium catalyst or (ii) in the presence of a metallic catalyst and water to yield a corresponding dialdehyde.

2. A process according to claim 1, wherein said 1,4-diol, 1,5-diol, or 1,6-diol is a compound shown by the following formula (1), (2), or (3): 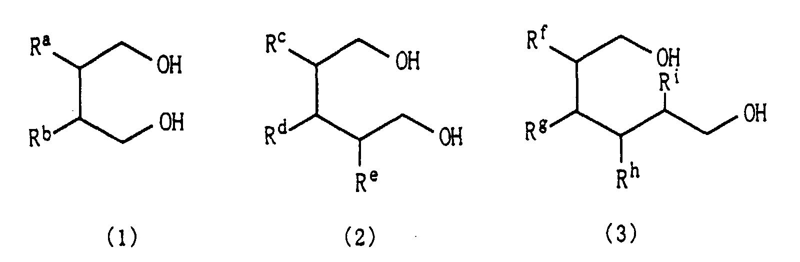 wherein each of R^{a} and R^{b} in the formula (1) is, identical to or different from each other, a hydrogen atom, a hydroxyl group which may be protected by a protective group, a hydrocarbon group, or a heterocyclic group, where R^{a} and R^{b} may be combined to form a double bond or to form a ring with the adjacent two carbon atoms; each of R^{c}, R^{d}, and R^{e} in the formula (2) is, identical to or different from one another, a hydrogen atom, a hydroxyl group which may be protected by a protective group, a hydrocarbon group, or a heterocyclic group, where at least two of R^{c}, R^{d}, and R^{e} may be combined to form a double bond, or to form a ring with the adjacent carbon atoms; and each of R^{f}, R^{g}, R^{h}, and Rⁱ in the formula (3) is, identical to or different from one another, a hydrogen atom, a hydroxyl group which may be protected by a protective group, a hydrocarbon group, or a heterocyclic group, where at least two of R^{f}, R^{g}, R^{h}, and Rⁱ may be combined to form a double bond or to form a ring with the adjacent carbon atoms.

3. A process according to claim 2, wherein R^{a} and R^{b} in the formula (1) are combined to form, with the adjacent carbon atoms, an aromatic carbon ring or an aromatic heterocyclic ring which may have a substituent on a ring.

4. A process according to claim 2, wherein the compound shown by the formula (1) is o-benzenedimethanol.

5. A process according to claim 1, wherein said ruthenium catalyst is at least one selected from a metallic ruthenium, a ruthenium oxide, a perruthenic acid or its salt, and a ruthenium complex.

6. A process according to claim 1, wherein said ruthenium catalyst is a carrier-supported ruthenium catalyst comprising a catalytic component supported on a carrier.

7. A process according to claim 1, wherein said metallic catalyst is at least one catalyst selected from a catalyst containing an element of Groups 5 to 11 of the Periodic Table of Elements, and a catalyst comprising a heteropolyacid or its salt.

8. A process according to claim 1, wherein said metallic catalyst is a carrier-supported metallic catalyst comprising a catalytic component supported on a carrier.

9. A process according to claim 1, wherein a reaction is performed under neutral or acidic conditions.

10. A process according to claim 1, wherein water is added to a reaction system, or a reaction is performed without removing water out of the reaction system, which water is by-produced during the reaction.

11. A process according to claim 1, wherein a reaction system includes 0.01 to 10 moles of water relative to 1 mole of the material diol.
